Europäisches Patentamt

European Patent Office

Office européen des brevets

(11) Publication number: **0 370 158**
**A1**

# EUROPEAN PATENT APPLICATION

(21) Application number: **89100900.3**

(22) Date of filing: **19.01.89**

(51) Int. Cl.⁵: **A61M 25/00**

(30) Priority: **25.10.88 US 261970**

(43) Date of publication of application:
**30.05.90 Bulletin 90/22**

(84) Designated Contracting States:
**DE FR GB IT**

(71) Applicant: **Martin, Geoffrey S.**
**159 Donnelly Drive**
**Mississauga Ontario L5G 2M3(CA)**

(72) Inventor: **Martin, Geoffrey S.**
**159 Donnelly Drive**
**Mississauga Ontario L5G 2M3(CA)**

(74) Representative: **Lewis, Samuel Hewitt et al**
**FORRESTER & BOEHMERT**
**Widenmayerstrasse 4/I**
**D-8000 München 22(DE)**

(54) **Catheter for prolonged access.**

(57) A flexible dual lumen catheter is provided for use especially in prolonged access for haemodialysis treatments and includes an elongate body (22) having side-by-side lumens (44, 46). Connecting tubes are attached to the lumens. The body has two portions, a distal portion for engagement within body tissue and a proximal portion extending between the connecting tubes and the distal portion. The distal portion has minimal rigidity for acceptance in the body tissue and the proximal portion has greater rigidity sufficient to minimize the risk of kinking during insertion and during normal use. A guide is also provided for use with the catheter. The guide has at least one weakness strip (74) extending longitudinally to permit the guide to be torn for removal from the catheter after insertion.

EP 0 370 158 A1

This invention relates to a dual lumen catheter and more particularly to such a catheter for longer term insertion into a vein of a patient for use in haemodialysis treatments.

Dual lumen catheters have been available for many years for a variety of medical purposes. It is only in recent years, however, that such catheters have been developed for use in procedures such as haemodialysis. The general form of dual lumen catheters goes back to as early as 1882 when Pfarre patented such a catheter in the United States under Serial No. 256,590. This patent teaches a flexible dual lumen catheter which is used primarily for cleaning and drainage of, for example, the bladdar, rectum, stomach and ear. In this type of catheterization, the catheter is introduced into an existing body orifice without the use of a guiding structure.

More recently a dual lumen catheter was developed and patented by Blake et al under U.S. Patent No. 3,634,924 for a different purpose. This 1972 patent teaches a dual lumen cardiac balloon catheter which is introduced into a large vein and the balloon inflated to control the flow in the vein. The catheter can in fact be placed by using the balloon as a "sail" to move with the blood from an ante-cubital or other peripheral vein through for example, the right heart chambers into the smaller radicals of the pulmonary artery where the catheter takes up its intended function. This patent is of interest because it explains how to make a tip for a dual lumen structure of the type which has become common for a variety of purposes including haemodialysis. The structure uses a plug to seal the end of one lumen and a wire which retains the shape of the other lumen during formation of the tip in a heated die.

The surgical cut-down technique was used universally for vascular catheter access and this technique can be traced back to the 17th century. It is only as recently as about 1952 that a new approach to vascular access was taught when an article was published by Dr. Sven Ivar Seldinger resulting from a presentation made at the Congress of the Northern Association of Medical Radiology at Helsinki in June of 1952. The technique is still current and essentially involves the use of a hollow needle to make an initial puncture, a very flexible wire is then entered through the needle and positioned in the vessel, and then the needle is withdrawn and a catheter is entered percutaneously over the wire which is itself later withdrawn. With this technique it became possible to make less traumatic vascular access and this has now become an accepted method of performing access in numerous medical techniques. One of these techniques which has been the subject of much research and development is haemodialysis.

Haemodialysis can be defined as the temporary removal of blood from a patient for the purpose of extracting or separating toxins therefrom and the return of the cleansed blood to the same patient. Haemodialysis is indicated in patients where renal impairment or failure exists, that is, in cases where the blood is not being properly or sufficiently cleansed, (particularly to remove water) by the kidneys.

In the case of chronic renal impairment or failure, haemodialysis has to be carried out on a repetitive basis. For example, in end stage kidney disease where transplantation of kidneys is not possible or for medical reasons is contra-indicated, the patient will have to be dialysed about 100 to 150 times per year. This can result in several thousand accesses to the blood stream to enable the active haemodialysis to be performed over the remaining life of the patient.

Towards the end of 1960, Dr. Stanley Shaldon and colleagues developed, in the Royal Free Hospital in London, England, a technique for haemodialysis by percutaneous catheterization of deep blood vessels, specifically the femoral artery and vein. The technique was described in an article published by Dr. Shaldon and his associates in the October 14th, 1961 edition of The Lancet at Pages 857 to 859. Dr. Shaldon and his associates developed single lumen catheters having tapered tips for entry over a Seldinger wire to be used in haemodialysis. Subsequently, Dr. Shaldon began to insert single lumen inlet and outlet catheters in the femoral vein and this was reported in the British Medical Journal of June 19th, 1963. The purpose of providing both inlet and outlet catheters in the femoral vein was to explore the possibility of a "self-service" approach to dialysis. Dr. Shaldon was subsequently successful in doing this and patients were able to operate reasonably normally while carrying implanted catheters which could be connected to haemodialysis equipment periodically.

Some use was made of a flexible dual lumen catheter inserted by surgical cut-down as early as 1959. An example of such a catheter is that of McIntosh and colleagues which is described in the Journal of the American Medical Association of February 21, 1959 at pages 137 to 138. In this publication, a form of dual lumen catheter is made of non-toxic vinyl plastic and described as being inserted by cut-down technique into the saphenous vein to the inferior vena cava.

The advantage of dual lumen catheters in haemodialysis is that only one vein access need be affected to establish continued dialysis of the blood, because one lumen serves as the conduit for blood flowing from the patient to the dialysis unit and the other lumen serves as a conduit for treated blood returning from the dialysis unit to the patient. This contrasts with prior systems where either two insertions were necessary to place the two catheters as was done by Dr. Shaldon, or a single catheter was used with a complicated dialysis machine which alternately removed blood and returned cleansed blood through a single-lumen.

The success of Dr. Shaldon in placing catheters which remained in place for periodic haemodialysis caused further work to be done with different sites. Dr. Shaldon used the femoral vein and in about 1977 Dr. P.R. Uldall, in Toronto Western Hospital, Canada, began clinical testing of a subclavian catheter that would remain in place between dialysis treatments. An article describing this was published jointly by Dr. Uldall, the present inventor, and others in Dialysis and Transplantation, Volume 8, No. 10, in October 1979. Subsequently Dr. Uldall began experimenting with a coaxial dual lumen catheter for subclavian insertion and this resulted in Canadian Patent No. 1,092,927 which issued on January 6, 1981. Although this particular form of catheter has not achieved significant success in the market-place, it was the forerunner of dual lumen catheters implanted in the subclavian vein for periodic haemodialysis.

The next significant step in the development of a dual lumen catheter for haemodialysis is Canadian Patent No. 1,150,122 to the present inventor. This catheter avoided the disadvantages of the Uldall structure and achieved some commercial success.

A subsequent development is shown in U.S. Patent No. 4,451,252 also to the present inventor. The structure shown in this patent utilizes the well known dual lumen configuration in which the lumens are arranged side-by-side separated by a diametric septum. The tip is formed to make it possible to enter a Seldinger wire through one of the lumens and to use this wire as a guide for inserting the catheter percutaneously. This type of structure is also shown in a European Patent Application to Edelman published under No. 0 079 719, and in U.S. Patents Nos. 4,619,643, 4,583,968, 4,568,329, 4,543,087, 4,692,141, 4,568,329, and U.S. Design Patent No. 272, 651. All of these structures require quite stiff tips to facilitate dilating body tissue as the catheter is pushed over the wire and yet they must have flexible bodies to allow the catheter to follow the contour of the vein after placement. This has led to several approaches to manufacture a suitable tip on the body. Firstly the body material is chosen as a compromise between strength for end loading during insertion, flexibility after insertion, and resistance to kinking in the part of the catheter exposed after the catheter is in place. The tip is then formed from the same material and stiffened by concentrating the material, or a separate tip is added using stiffer material, or the tubing is first given a plug which stays in the tip after formation. Clearly the major design criteria are its need for strength to insert the catheter and the conflicting need for softness and flexibility after insertion.

The result of the design requirements for Seldinger placement is that the types of catheters satisfying these requirements are not suitable for prolonged placement. Catheters for this use must be extremely flexible to avoid stressing the vein and also to permit the catheter to move in the flow of blood to minimize the possibility of the catheter remaining in pressure contact with the wall of the vein at one spot for prolonged periods.

In summary, although there have been significant developments in the structures of dual lumen catheters, these structures are limited in their usefulness primarily because of the very design criteria inherent in making them suitable for placement by the Seldinger technique. The catheters are not suitable for prolonged placement.

The requirements for prolonged placement also lead to difficulties. While a soft and flexible catheter would be acceptable insofar as it follows vein contours and has minimal resistance to deflection in the flow of blood, such a catheter would be prone to kinking and flexing in the part positioned outside the patient. Also such flexibility would make handling during insertions impractical. As a result there is a tendency to compromise the design resulting in less than ideal characteristics for the tip and the portion of the catheter located in the vein.

It is among the objects of the present invention to provide a catheter for prolonged placement which mitigates the disadvantages of prior art structures.

In one of its aspects the invention provides a flexible dual lumen catheter for use in procedures such as haemodialysis, the catheter comprising:
an elongate body defining side by side lumens and having distal and proximal ends, one of the lumens being an intake lumen for receiving body fluid and the other being a return lumen for leading fluid to the body;
a pair of connecting tubes attached one to the intake and one to the return lumen;
a tip adjacent said distal end, the tip defining an intake opening spaced from the distal end of the body to provide access to the intake lumen, and a return opening leading from the return lumen at the distal end of the body; and
the body being in two portions, a distal portion adjacent the distal end for engagement within body tissue and a proximal portion extending between the connecting tubes and the distal portion, the distal portion having minimal rigidity for acceptance in the body tissue and the proximal portion having greater rigidity sufficient to minimize the risk of kinking during insertion and during normal use.

In another of its aspects the invention provides a method of making a flexible catheter especially for use in haemodialysis when prolonged access is indicated, the method comprising the steps:

providing an elongate first part having proximal and distal ends and a cross-section defining intake and return lumens in side by side relationship, the material being of a very flexible medical grade thermoplastic synthetic plastic material;

forming a tip adjacent the distal end of the first part to provide access to the intake lumen at a point spaced from the distal end and an exit from the return lumen at said distal end;

providing a shorter second part adapted to slip snugly over the first part to cover a proximal portion of the first part extending from the proximal end of the first part, the second part being of a medical grade thermoplastic synthetic plastic material;

providing a pair of connecting tubes of thermoplastic material;

engaging the second part over the proximal portion of the first part;

placing ends of the tubes in the respective intake end and return lumens;

supporting the lumens internally and applying heat and pressure to melt and deform the tubes and first and second parts to create a connection between the tubes and the first and second parts;

whereby the resulting catheter has said proximal portion and a distal portion between the proximal portion and said distal end, the proximal portion being stiffer than the distal portion.

In yet another of its aspects the invention provides a catheter and guide assembly as claimed in Claim 10 in which the handle means comprises a pair of opposed projections extending outwardly from the tubular main part.

These and other aspects of the invention will be better understood with reference to the drawings and associated disclosure, in which:

Fig.1 is a side view of a catheter according to a preferred embodiment of the invention, with the distal end turned for a more perspective view of a tip on the catheter;

Fig. 2 is a sectional end view on line 2-2 of Fig. 1 and drawn to a larger scale;

Fig. 3 is a view similar to Fig. 2 and drawn on line 3-3 of Fig. 1;

Fig. 4 is a sectional side view to the same scale as Figs. 2 and 3 and drawn on line 4-4 of Fig. 1 to show a portion of the catheter at this location;

Fig. 5 is a sectional side view of the attachment between connecting tubes and the body of the catheter;

Fig. 6 (drawn beside Fig. 1) is a side view of a guide for use in placing the catheter in position in a patient;

Fig. 7 is a sectional end view on line 7-7 of Fig. 6; and

Fig. 8 is a view showing the manipulation of the guide to tear it for separation from the catheter.

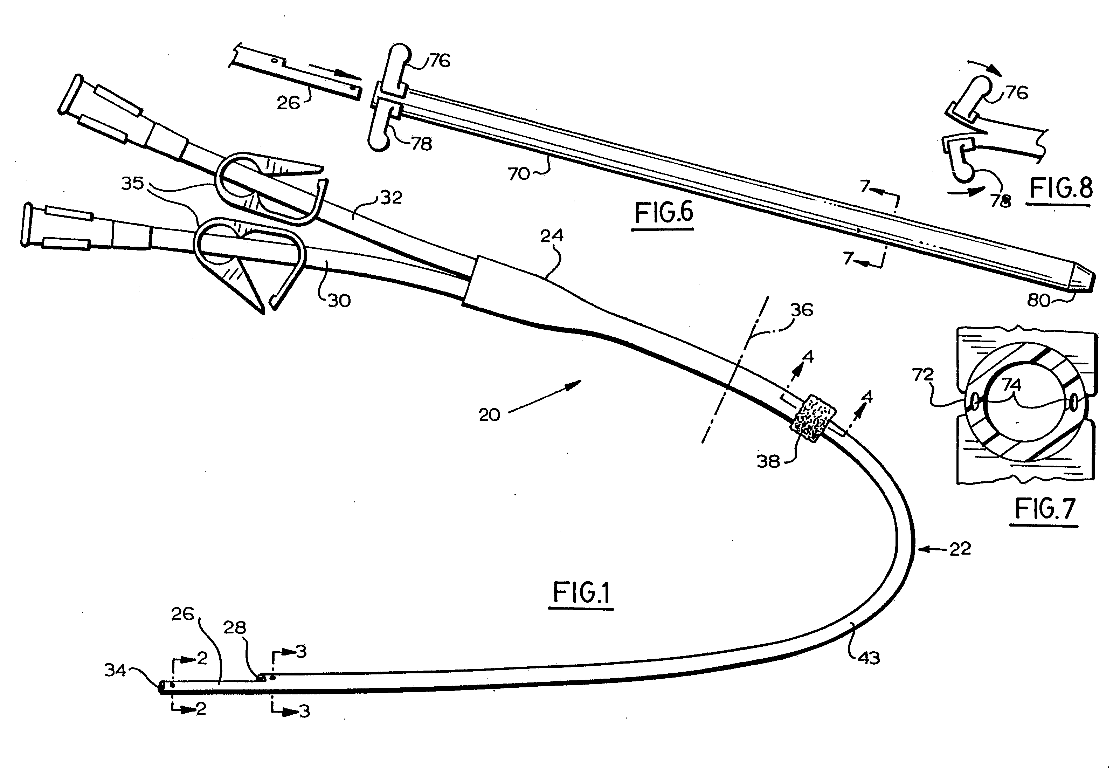

Reference is made firstly to Fig. 1 which illustrates an exemplary haemodialysis catheter 20 having an elongate main body 22 which extends from a connection 24 to a tip 26 and which contains intake and return lumens as will be described. In use, body fluid is withdrawn via an intake opening 28 at tip 26 into the intake lumen in the body 22 which is connected at the connection 24 to an intake tube 30 to withdraw blood. Treated blood is then returned through a return tube 32 via the return lumen which terminates at a return opening 34 at the distal end of the body 22.

Conventional clamps 35 are provided on the tubes 30, 32 which are of course flexible to permit use of these clamps. When engaged in a patient, the catheter would normally be engaged to a point indicated in the ghost outline by the chain-dotted outline 36 and a fibrous cuff 38 is provided to provide a seal where blood would clot in the cuff and seal the catheter to the patient.

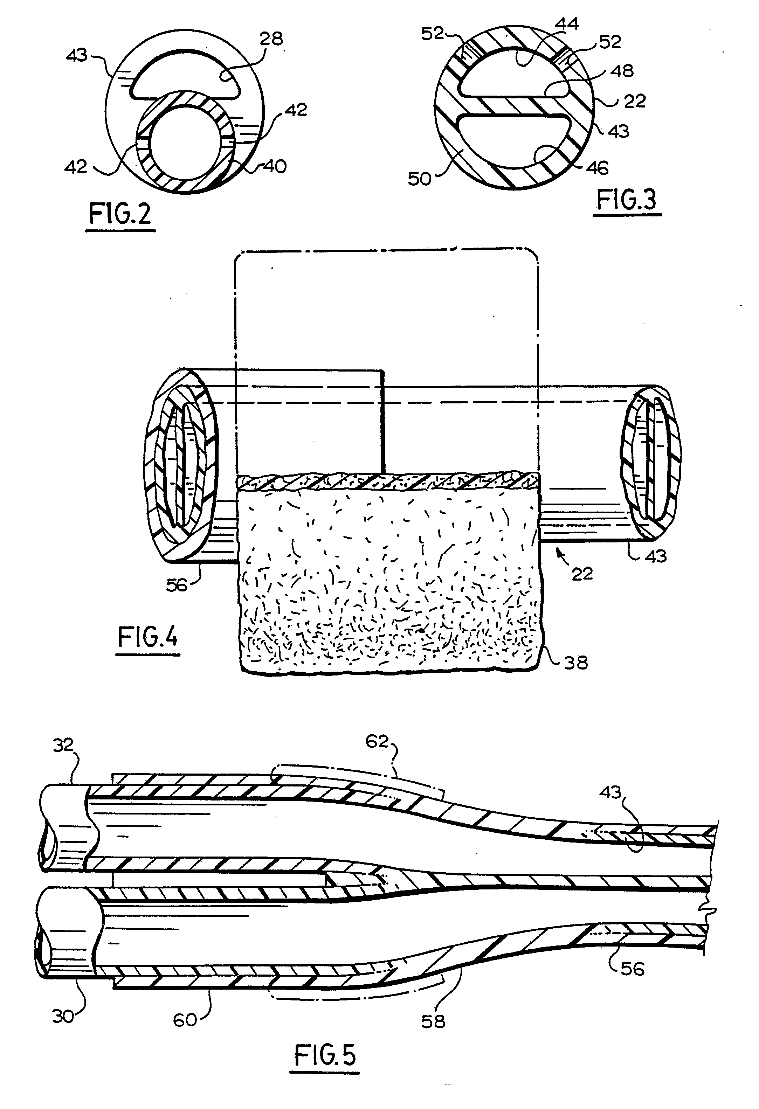

The form of the tip immediately adjacent the distal end of the body 22 is better seen in Fig. 2. This sectional view shows a cylindrical tubular end portion 40 having a pair of side openings 42 to complement the return opening 34 (Fig. 1) and blending into the main body 22. In the background can be seen opening 28 which is simply the end of the intake lumen. The end portion 40 is formed from an extrusion the shape of which is seen in Fig. 3. This extrusion extends throughout the body 22 as will be explained, but is exposed only between the cuff 38 and the tip 26. The extrusion is in other words a first part of the body and will be designated by numeral 43. It will be seen that this part of the body is essentially cylindrical although within the practical limitations of extruding, this shape may vary slightly.

The part 43 contains a D-shaped intake lumen 44 and a similarly shaped return lumen 46. These D-shaped lumens are separated by a septum 48 extending diametrically across the body between opposed locations on the inside of a side wall 50. The intake opening 28 is supplemented by a pair of side openings 52 adjacent the opening 28 and spaced circumferentially about the wall 50.

The section of the first part of the body shown in Fig. 3 actually extends throughout the length of the body 22 but is complemented by a stiffening second part extending between the proximal end of the body at the connector 24 and the cuff 38. It will be seen in Fig. 1 that this part of the body is wider than the Part between the cuff 38 and the tip and the structure of this part will be described in more detail with reference to Figs. 4 and 5.

Returning to Fig. 1, the tip 26 is formed by first cutting off a section of the body 43 corresponding to the intake lumen while leaving the septum and return lumen intact. A round mandrel is then placed in the portion between openings 28 and 34 and heat is applied using a shaped die to permanently deform the material into the tubular cylindrical tip 26. The complementary openings 42 and 52 are then machined using a hollow drill.

As seen in Fig. 4, the body 22 actually consists of the first part 43 which extends throughout the length of the body and a second part 56 which fits snugly about the first part between the cuff 38 and the connector 24. With reference to Fig. 4, the cuff 38 serves also to cover the junction or exposed end of part 56 so that this end will not cause any interference in use. At the other end of the part 56, and as seen in Fig. 5, this part extends about the internal or first part 43 and, for reasons which will be explained, blends into this part at a junction portion 58. Similarly, the tubes 30, 32 also blend into this portion where they engage into the D-shaped lumens of the first part 43.

The arrangement shown in Fig. 5 has become typical in the art. Initially the D-shaped lumens are flared to receive the tubes 30, 32 and the part 56 is shaped with a flare so that it will extend over the junction between the tube and the part 43. Generally the parts have the shape shown in Fig. 5. Next shaped heating tools are used in conjunction with shaped rods which are positioned inside the structure and the heat is applied externally under moderate pressure to cause some deformation and melting of the material so that the aforementioned blending takes place to attach the parts to one another and also to seal the joint. As a result, there is some of the part 56 extending over the tubes at a portion indicated by numeral 60. This portion is optional but does serve to protect the tubing during the heating process. After assembly, the portion can be turned back on itself as indicated in ghost outline and indicated by numeral 62 to further enhance the connection and strengthen the structure. However this is a optional step which is not essential to the operation of the catheter.

It will now be apparent that the body 22 is really in two portions as far as its characteristics are concerned. A distal portion between the cuff 38 and the tip is very flexible and after insertion is capable of taking up the contour of a vein and possibly moving in the vein as a result of blood flow and the forces applied in this flow. There is therefore less likelihood that the tip will stay in one position in a vein where it could, during use, cause stagnation points in the blood between the catheter and the vein. To help increase the likelihood of movement during use, the side openings 42 and 52 are provided in the structure as well as the axially orientated intake opening 28 and return opening 34.

The portion between the cuff 38 and the proximal end of the body 22 is stiffer because of the inclusion of the second part 54 of the body in external engagement around the first part 43 containing the D-shaped lumens. This increases the cross-sectional area and enhances the stiffness of the body and diminishes the likelihood of kinking caused by manipulation and accidental movement of the catheter outside the patient. The structure therefore overcomes the problems of providing a very flexible portion inside the patient and at the same time having sufficient rigidity outside the patient to minimize the risk of kinking.

The material used for the body of the catheter is typically soft medical grade polyurethane, with a Durometer A reading of 80. The portion of the body inserted in the patient is typically 11.5 French for haemodialysis and the insertion length 19 cm. The cuff is preferably of fibrous DACRON (trade mark of E. I. Dupont for a polyester material).

As mentioned previously, the catheter would often be inserted using conventional cut-down techniques. However, a modified Seldinger technique can also be used. In this case the Seldinger wire is used to lead a tubular guide into position in a vein and then the catheter is fed into the guide. It is then necessary to remove the guide by tearing or otherwise "peeling" the guide off the catheter. The present invention also includes a guide suitable for such use.

As seen in Fig. 6, a guide is provided and indicated generally by the numeral 70. A catheter tip 26 is seen adjacent the guide to illustrate how the catheter is engaged in the guide during use. As seen in Figs. 6 and 7, the guide is tubular having a wall 72 which has longitudinal diametrically opposed small openings 74 forming weakness strips in the wall to facilitate preferential tearing axially of the guide. The proximal end of the guide has a pair of handles 76, 78 extending in opposition to one another so that they can be rotated in the manner shown in Fig. 8 to initiate tearing of the guide at the weakness strips. This tearing can continue simply by pulling the handles apart. The tearing will run the full length of the guide and through the slightly conical tip 80. This facilitates removal of the guide by simply tearing it apart and taking it away from the catheter leaving the catheter in place. The guide will of course only receive the part of the catheter extending between the cuff 38 (Fig. 1) and the distal end of the catheter and will be proportioned to receive this part snugly in sliding relationship.

The weakness strips 74 are built internally in the wall 72 and can be formed by the inclusion of a material during the extrusion process. For instance nylon threads could be used or simple material which in itself would be inherently weak and would diminish the strength of the wall in that location. In the event that threads are used, they would be engaged in the extrusion and material chosen such that there was no bonding between the wall and the thread to ensure that there is a weakness strip in this location.

The outside diameter of the guide is advantageously the same as that of the part 56 (Fig. 5) of the catheter so that when the catheter is in place the catheter will seal the entry site where the guide was entered.

The foregoing exemplary description teaches the invention in preferred form and is not to be used to limit the generality of the invention as claimed.

The features disclosed in the foregoing description, in the claims and/or in the accompanying drawings may, both, separately and in any combination thereof, be material for realising the invention in diverse forms thereof.

## Claims

1. A flexible dual lumen catheter for use in procedures such as haemodialysis, the catheter comprising
an elongate body (22) defining side by side lumens (44, 46) and having distal and proximal ends, one of the lumens being an intake lumen for receiving body fluid and the other being a return lumen for leading fluid to the body and the catheter being characterised by
a pair of connecting tubes (30, 32) attached one to the intake and one to the return lumen;
a tip (26) adjacent said distal end, the tip defining an intake opening (28) spaced from the distal end of the body to provide access to the intake lumen, and a return opening (34) leading from the return lumen at the distal end of the body; and being characterised in that
the body (22) is in two portions, a distal portion adjacent the distal end for engagement within body tissue and a proximal portion extending between the connecting tubes and the distal portion, the distal portion having minimal rigidity for acceptance in the body tissue and the proximal portion having greater rigidity sufficient to minimize the risk of kinking during insertion and during normal use.

2. A catheter as claimed in Claim 1 in which the lumens are substantially D-shaped in cross-section.

3. A catheter as claimed in Claim 1 or Claim 2 in which the proximal portion has a greater cross-sectional area than the distal portion.

4. A catheter as claimed in Claim 1 or Claim 2 in which the elongate body consists of a first part (43) forming the distal portion and having a hidden portion extending through the proximal portion, and a second part (56) positioned about and in close proximity with the hidden portion so that the proximal portion has a greater cross-sectional area than the distal portion.

5. A catheter as claimed in Claim 2 in which the body includes an end portion (40) between the intake and outlet openings, the end portion being substantially cylindrical and being tubular for carrying the fluid returning the body.

6. A catheter as claimed in Claim 2 or Claim 5 in which the intake opening (28) is at the distal end of the intake lumen (44).

7. A catheter as claimed in Claim 2 in which the intake and return lumens (44, 46) further define side openings (42, 52) adjacent respective intake and return openings.

8. A catheter as claimed in Claim 2 in which the tip (26) is formed integrally with the elongate body (22).

9. A catheter as claimed in Claim 4 in which the second part (56) has an extension projecting over a portion of the tubes (30, 32) adjacent the body.

10. A catheter as claimed in any one of Claims 2 to 9 and further including a fibrous cuff (38) about the body at the junction of the proximal and distal portions of the body.

11. A catheter and guide assembly for use in procedures such as haemodialysis, comprising:
a catheter as claimed in any preceding Claim,
a guide (70) adapted to receive at least part of the distal portion of the catheter and including the tip (26), the guide including a tubular main part proportioned to contain the catheter body (22) snugly for relative sliding movement of the catheter and guide, with the catheter entered into the guide at a proximal end of the guide for movement in the guide towards the distal end of the guide, and handle means (76, 78) projecting from a proximal end of the guide, the tubular main part of the guide having at least one weakness strip (74) built internally into the wall of the tubular main part whereby manipulation of the handle means will cause a tear to develop longitudinally along the weakness strip to open the main part for removal off the catheter after catheter insertion.

12. A catheter and guide assembly as claimed in Claim 9 in which the handle means comprises a pair of opposed projections (76, 78) extending outwardly from the tubular main part.

13. A catheter and guide assembly for use in procedures such as haemodialysis, comprising:
a catheter as claimed in any one of claims 1 to 10

a guide adapted to receive at least part of the distal portion of the catheter body and including the tip (26), the guide including a tubular main part proportioned internally to contain the catheter body (22) snugly for relative sliding movement of the catheter and guide, with the catheter entered into the guide at a proximal end of the guide for movement in the guide towards the distal end of the guide, and proportioned externally to match the external measurement of the proximal portion of the catheter, including means (74) extending longitudinally of the guide to facilitate tearing the guide for removal off the catheter after catheter insertion.

14. An assembly as claimed in Claim 13 in which said distal and proximal portions of the body are substantially circular in cross-section.

15. A method of making a flexible catheter especially for use in haemodialysis when prolonged access is indicated, the method comprising the steps of:

providing an elongate first part (43) having proximal and distal ends and a cross-section defining intake and return lumens (44, 46) in side by side relationship, the material being of a very flexible medical grade thermoplastic synthetic plastics material;

forming a tip (26) adjacent the distal end of the first part to provide access to the intake lumen at a point spaced from the distal end and an exit from the return lumen at said distal end;

providing a shorter second part (56) adapted to slip snugly over the first part to cover a proximal portion of the first part extending from the proximal end of the first part, the second part being of a medical grade thermoplastic synthetic plastics material;

providing a pair of connecting tubes (30, 32) of thermoplastic material;

engaging the second part over the proximal portion of the first part;

placing ends of the tubes in the respective intake and return lumens;

supporting the lumens (44, 46) internally and applying heat and pressure to melt and deform the tubes (30, 32) and first and second parts (43, 56) to create a connection between the tubes and the first and second parts;

whereby the resulting catheter has said proximal portion and a distal portion betweeen the proximal portion and said distal end, the proximal portion being stiffer than the distal portion.

16. A guide for use with a catheter in procedures such as haemodialysis, the guide comprising a tubular main part proportioned to receive the catheter snugly for relative sliding movement of the catheter and guide with the catheter entered into the guide at a proximal end of the guide for movement in the guide towards the distal end of the guide, and handle means (76, 78) projecting from the proximal end of the guide, the tubular main part of the guide having at least one weakness strip (74) built internally into the wall (72) of this tubular main part whereby manipulation of the handle means will cause a tear to develop longitudinally along the weakness strip to open the main part for removal off the catheter after catheter insertion.

17. A guide as claimed in Claim 16 in which the handle means comprises a pair of opposed projections (76, 78) extending outwardly from the tubular main part.

18. In combination, a catheter and a tubular guide through which the catheter can be inserted, wherein the guide has a longitudinally extending weakness (74), along which the guide can be preferentially torn to facilitate removal of the guide from the catheter after insertion.

FIG.6

FIG.8

FIG.7

FIG.1

**FIG.2**

**FIG.3**

**FIG.4**

**FIG.5**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | US-A-4 692 141 (MAHURKAR) <br> * Whole document * | 1-9,11-12,13-14 | A 61 M 25/00 |
| Y | US-A-4 639 252 (KELLY et al.) <br> * Column 6, lines 30-41 * | 1-9 | |
| Y | CA-A-1 225 299 (BAI, CHAO-LIANG) <br> * Page 7, lines 8-16; page 34, lines 14-19 * | 1-9,11-12,13-14 | |
| Y | US-A-4 306 562 (OSBORNE) <br> * Abstract; figures 1-16 * | 11-12, 13-14 | |
| A | EP-A-0 185 977 (I-FLOW CORP.) <br> * Abstract; figure 1 * | 10 | |

TECHNICAL FIELDS
SEARCHED (Int. Cl.4)

A 61 M

The present search report has been drawn up for all claims

| Place of search | Date of completion of the search | Examiner |
|---|---|---|
| THE HAGUE | 31-01-1990 | MIR Y GUILLEN V. |

EPO FORM 1503 03.82 (P0401)